# EUROPEAN PATENT SPECIFICATION

(11) **EP 0 228 034 B1**
(45) Date of publication and mention of the grant of the patent: **03.03.1993**
(21) Application number: 86117631.1
(22) Date of filing: 18.12.1986
(51) Int. Cl.: A61K 31/195, A61K 31/155, C07C 279/04

(54) **Arginine and agmatine derivatives for treating protozoal infections**
Arginin- und Agmatinderivate zur Behandlung von Protozoen-Infektionen
Dérivés de l'arginine ou de l'agmatine pour le traitement des infections aux protozoaires

(30) Priority: 19.12.1985 US 811147
(43) Date of publication of application: 08.07.1987
(73) Proprietor: MERRELL DOW PHARMACEUTICALS INC., Cincinnati Ohio 45215-6300 (US)
(72) Inventor: Sjoerdsma, Albert, Cincinnati Ohio 45243 (US); McCann, Peter P., Cincinnati Ohio 45241 (US); Bey, Philippe, Cincinnati Ohio 45242 (US)
(74) Representative: Sgarbi, Renato

(56) References cited:
- EP-A- 0 094 886
- BE-A- 881 208
- BE-A- 881 209
- BE-A- 881 210
- GB-A- 2 078 735
- US-A- 4 134 918
- US-A- 4 139 563
- Biochemistry, vol.20 no.10, pages 3163-3166, American Chemical Society, Washington, US; A. Kallo et al.
- BIOCHEM.J., vol. 208, no. 2, 1982, pp. 435-441, The Biochemical Society, London, GB; A.J.BITONTI et al.
- Proc.Natl.Acad.Sci.USA, vol.84, pp.4278-4282, June 1987
- J. Protozool, vol. 28, no. 1, 1981, pp. 20-27, The Society of Protozoologists; C.J. Bacchi
- Tetrahedron Letters, vol. 25, no. 18, 1984, pp. 1891-1894, Pergamon Press Ltd., GB; P. Casara et al.

## Description

This invention relates to the use of certain irreversible enzyme inhibitors which interrupt the biosynthesis of polyamines and which inhibit the growth of certain protozoans responsible for the Chagas disease.

More specifically this invention relates to certain agmatine and arginine derivatives which are enzyme inhibitors useful in the treatment of animals suffering from Chagas disease.

Still more specifically, this invention relates to the use of X-substituted arginine and agmatine compounds having the formula 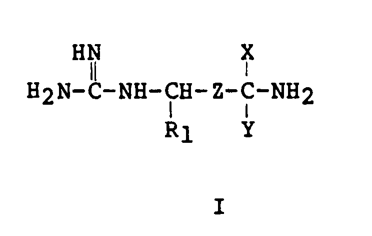
their pharmaceutically acceptable salts, their individual optical isomers and mixtures thereof, wherein R₁ is H or CH₃, Z is -CH₂-CH₂- or -CH=CH-, X is -CH₂F, -CHF₂, -CHCl₂, -CHClF,-C≡CH or -CH=C=CH₂, Y is H or COOR with R being H or C₁₋₁₈ alkyl, which compounds are enzyme inhibitors, for the manufacture of a medicament for the treatment of Chagas disease in animals, including man, said disease states being caused by infection of the animals with Trypanosoma cruzi. In those instances wherein Z is -CH=CH-, the double bond preferably is in its trans configuration, i.e. the (E) designated compound.

The invention also relates to certain novel compounds belonging to the above formula, which are defined in claims 7 to 10.

In essence the compounds depicted by formula I are halomethyl, acetylenic, or allenic derivatives of arginine and agmatine or their dehydro derivatives. For the most part these compounds are known and their preparation is adequately described in the prior art. In those specific instances wherein the compounds are novel, per se, such compounds may be prepared by methods and techniques analogously known in the art. For example, the halomethyl derivatives of the amines (i.e. Y is H and Z is -CH₂-CH₂-) may be prepared by the techniques of U.S. Patent 4134918, and the dehydro analogs thereof by using the techniques of British Patent No. 2083030 and with the techniques hereinbelow illustrated by Examples 1-3. The halomethyl derivatives of the α-amino acids (i.e. Y is -COOR) may be prepared by the teachings of South African Patent No. 78/3349 and their dehydro derivatives may be prepared by the teachings of British Patent No. 2083030. The acetylenic derivatives of the amino acids (i.e. Y is -COOR and X is -C≡CH) may be prepared by the teachings of U.S. Patent 4182891 and of the amines (i.e. Y is H and X is -C≡CH) may be prepared by the teachings of 4139563. The allenic derivatives of the compounds of formula I may be prepared from the acetylenic derivatives by the techniques of U.S. Patent 4,454,156 which are further elaborated upon by Casara in Tetrahedron Letters, Vol. 25, pg. 1891 (1984).

The technique for selectively converting an amino moiety to a guanidino moiety is illustrated by the following specific examples. This technique is generally available to make compounds of formula I from intermediates which are analogous to those used in these examples.

### EXAMPLE 1

### 1-Guanidino-4-amino-5-hexyne dihydrochloride

Triethylamine (4.5 ml, 30 mmoles) is added to a solution of 1,4-diamine-5-hexyne dihydrochloride (1.85 g, 10 mmoles) in water (50 ml) at -10°C. To this solution 3,5-dimethylpyrazole-1-carboxamidine nitrate (2.3 g, 10.5 mmoles) is slowly added (in small portions) and the resulting mixture is stirred 2 h at -10°C, and for 3 days at room temperature after all starting materials have reacted (this is controlled by electrophoresis and ninhydrin colorimetric analysis). Then, the mixture is acidified (pH 5-6) with a 1M solution of HCl and washed with dichloromethane (2 × 100 ml). The aqueous layer is concentrated under reduced pressure to a volume of 5 ml. This concentrated mixture is purified by ion exchange chromatography. (DOWEX® 50W-X8, 100-200 mesch, H⁺ Form) which is eluted by using a gradient of solution of HCl (0 to 4N). The eluted fractions are checked by electrophoresis on silica gel plates and colorimetric analysis (ninhydrin and Sakaguchi reagent). The fractions containing the guanidino material are concentrated together under reduced pressure to give the title compound (1.7 g) as a colorless oil. NMR(¹H) δ ppm: 1.76 (m, 4H); 2.96 (d,1H,J=1H₂); 3.16 (m, 2H); 4.2 (m, 1H). M/Z : MH⁺ 155; MH^{±} NH₃: 138; MH^{±} NH₂-CNHNH₂=98.

### EXAMPLE 2

### 1-Fluoro-2-amino-5-guanidinopentane dihydrochloride

Using the procedure similar to that described in the first example 1-fluoro-2,5-diaminopentane dihydrochloride (1.93 g, 10 mmoles) afforded 1.9 g of the title compound, mp: 158°C after recrystallization from ethanol/ether.

Similarly, using the procedure similar to that described in the first example, 4,7-diamino-1,2-heptadiene dihydrochloride (2.0 g, 10 mmole) afforded 2.1 g of 4-amino-7-guanidino-1,2-heptadiene dihydrochloride as an oil.

### EXAMPLE 3

### (E)-α-Fluoromethyl-3,4-dehydro arginine monohydrochloride

Using the procedure similar to that described in example one (E)-α-fluoromethyl-3,4-dehydro ornithine monohydrochloride (1.8 g, 10 mmole) in 50 ml of water, triethylamine (3 ml, 20 mmoles) and 3,5-dimethylpyrazole-1-carboxamidine nitrate (2.5 g, 1.1 mmoles) yielded, after two weeks at room temperature, 2.2 g of the title compound as a white solid, which was recrystallized from absolute EtOH (mp 171°C, decomp.). H¹NMR Data: (D₂O, DCl) (¹H) δ (ppm) 4.0 (d, 2H); 4.65 (q, 1H); 5.25 (q, 1H); 5.75 (m, 2H).

Using the procedure similar to that described in example three, α-fluoromethyl ornithine monohydrochloride (2.0 g, 10 mmoles) afforded 2.05 g of α-fluoromethyl arginine monohydrochloride, mp 190°C after recrystallized from absolute EtOH.

In a similar manner, by following the teachings set forth above in examples 1-3 and by following the teachings set forth in the above cited prior art the following compounds may also be prepared:
α-difluoromethyl-3,4-dehydro arginine;
α-fluoromethyl-2,3-dehydro agmatine;
α-difluoromethyl-2,3-dehydro agmatine;
α-acetylenic-3,4-dehydro arginine;
α-acetylenic-2,3-dehydro agmatine;
α-allenyl-3,4 dehydro arginine; and
α-allenyl-2,3-dehydro agmatine.

Illustrative examples of the salts of the compounds of this invention include non-toxic acid addition salts formed with inorganic acids, such as, hydrochloric, hydrobromic, sulfuric and phosphoric acid, and organic acids, such as, methanesulfonic, salicyclic, maleic, malonic, tartaric, citric, cyclamic and ascorbic acids. In addition to the salts indicated above, the term salts is taken to include those internal salts or zwitter-ions of those compounds of formula (I) above that are amphoteric in nature. Moreover, whereas the optical configuration for the compounds described herein is not specifically designated, it is recognized that the α-carbon atom possesses an asymmetric center and that individual optical isomers of these compounds exist. Accordingly, both the D- and L-optical isomers as well as the racemic mixtures are contemplated as being within the scope of this invention.

As stated above, the derivatives of arginine and agmatine of formula I are irreversible inhibitors which are capable of interrupting the biosynthesis of polyamines and which are now found to be useful in the treatment of diseases caused by parasitic infections with certain protozoa.

The rationale of polyamine metabolism has been suggested by Cohen, Science, 205, p 964 (1974). Impairment of the biosynthesis of polyamines by means of enzyme inhibitors also has been known to cause a decrease in cell proliferation in animals. Although the physiological role of the polyamines has not yet been clearly delineated, some evidence suggests their involvement with cell division and rapid cell growth. (H.S. Williams-Ashman et al., The Italian J. Biochem. 25, 5-32 (1976), A. Raina and J. Janne, Med. Biol. 53, 121-147 (1975) and D.H. Russell, Life Sciences 13, 1635-1647 (1973)). Indeed, there is evidence suggesting that polyamines are associated with both normal and rapid proliferative mammalian cell growth; their being an increase in both the synthesis and accumulation of polyamines following a stimulus causing cell growth.

Polyamines are also known to be essential growth factors in non-mammalians, particularly in such microorganisms as, for example, E. Coli, Enterobacter, Klebsiella, Staphylococcus aureaus, C. cadaveris, Salmonella typhosa and Hemophilus parainfluenzae.

It is further known by the teachings of U.S. Patent 4399151 that the growth of certain protoza has been inhibited with ornithine decarboxylase inhibitors. Indeed, the ornithine decarboxylase inhibitors have been shown to be useful in the treatment of African sleeping sickness (caused by Trypanosoma brucei rhodesiense and Trypanosoma brucei gambiense) and in the treatment of coccidiosis (caused by Eimeria tenella). Quite surprisingly, these same ornithine decarboxylase inhibitors were found to be ineffective in the treatment of other diseases caused by infection with other Trypanosoma. Notable amongst these disease states not controlled by ornithine decarboxylase inhibitors are Chagas disease and coccidiosis caused by species of Eimeria other than tenella.

The protozoan parasite Trypanosoma cruzi is the etiologic agent of Chagas disease which is endemic to vast areas of South and Central America. It is a particularly dreadful disease not only because of the severe acute and chronic debilitating symptoms suffered by millions, but also because there is no known effective treatment. Thus, the use of the compounds of formula I in the prevention and treatment of the disease and in the control of the causative parasite is, indeed, a significant advance.

In the use of the compounds of this invention as anti-Chagas disease agents, the relative potencies of the enzyme inhibitory properties may readily be determined by standard in vitro laboratory techniques. Standard in vivo evaluations may also be effected (however, because of the lack of an effective chemotherapeutic agent to control the protozoa, difficulty is experienced in finding suitable laboratories in the United States which routinely conduct such procedures). However, based on the early data, it is expected that effective treatment is about 0.5 g to 5 g per kilogram of body weight per day. In those particularly severe cases, because of the relative low toxicity of these compounds, it is expected that initial therapy may be as high as 8 g per kilogram of body weight per day. Treatment most preferably is with 2% aqueous formulations, preferably by infusion at early treatment stages and by standard pharmaceutical formulations at a later stage.

In addition to the foregoing methods of treating "patients" suffering from the disease states caused by Trypanosoma cruzi, it is also advantageous to control the spread of the disease with prophylactic techniques. For example, not only has the spread of Chagas disease been associated with the metacyclic tryptomastigotes present in the fecal fluids of insect vectors, but also through the blood administered in blood transfusions. Thus, the patients receiving blood transfusions may receive prophylactic treatment but also the blood, per se, may be pre-treated with the compounds of formula I in order to eliminate this source of parasitic infection.

Another way to advantageously administer compounds of this invention is to administer combinations of the compounds of this invention, preferably administering one member of the group of compounds defined by formula I wherein Y is other than H, and another member wherein Y is hydrogen, i.e. a combination of an arginine derivative and an agmatine derivative.

The compounds of formula I may be formulated for use as anti-protozoal agents according to standard prior art teachings. Preferably use of physiological solutions having about 2% of the compounds of formula I may be useful for infusion, or more concentrated solutions may be used in drinking water. Other formulations, the preparation of which is well known in the art, may be used.

As is true for most classes of compounds found useful as chemotherapeutic agents certain sub-classes and certain species are preferred. In this instance those compounds wherein X represents a halomethyl (mono- or di-) are most preferred, those compounds wherein Z is either saturated or unsaturated are preferred, those compounds wherein Y is a COOH moiety are preferred over their ester derivatives. Conjunctive therapy with the preferred arginine derivatives and the preferred agmatine derivatives is also preferred. A preferred X and/or Z- modified arginine or agmatine compound is also preferred. Specifically preferred species are:
α-fluoromethyl arginine and its 3,4-dehydro analog,
α-difluoromethyl arginine and its 3,4-dehydro analog,
α-fluoromethyl agmatine and its 2,3-dehydro analog,
α-difluoromethyl agmatine and its 2,3-dehydro analog,
α-acetylenic agmatine,
α-acetylenic arginine and
α-allenyl agmatine.

## Claims (Claims for the following Contracting State(s): BE, CH, DE, FR, GB, IT, LI, LU, NL, SE)

1. Use of a X-substituted arginine or agmatine of the formula 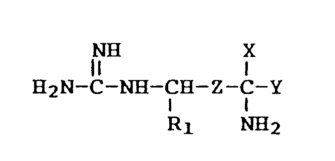 and the pharmaceutically acceptable salts thereof, the individual and racemic mixtures of their optical isomers, wherein
R₁ is H or CH₃,
Z is -CH₂-CH₂- or -CH=CH-,
X is -CH₂F, -CHF₂, -CHCl₂, -CHClF, -C≡CH or -CH=C=CH₂,
Y is H or -COOR with R being H or (C₁₋₁₈)alkyl for preparing a medicament for treating Chagas disease.

2. Use of a compound according to claim 1 wherein X is halomethyl.

3. Use of a compound according to claim 1 wherein X is fluoromethyl.

4. Use of a compound according to claim 1 wherein X is difluoromethyl.

5. Use of a compound according to claim 1 which is an arginine derivative.

6. Use of a compound according to claim 1 which is an agmatine derivative.

7. Compounds of the formulae 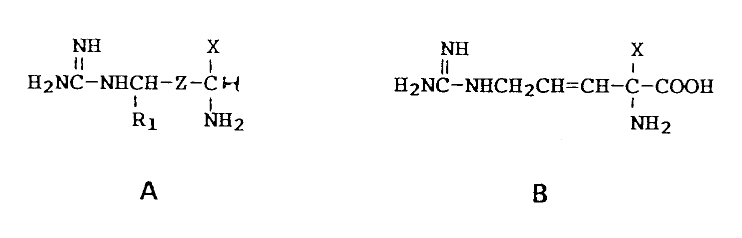 the pharmaceutically acceptable salts thereof, the individual optical isomers and mixtures thereof, wherein X is -CH₂F, -CHF₂, and -CH=C=CH₂,
Z is -CH₂-CH₂- or -CH=CH-,
and R₁ is hydrogen, with the proviso that when Z is -CH₂CH₂-, X is -CH=C=CH2.

8. A compound of claim 7 wherein X is -CH₂F or -CHF₂.

9. A compound of claim 7 wherein X is -CH=C=CH₂.

10. Compounds of claim 7 wherein the double bond is in its trans configuration.

11. A process preparing a compound of the formulae 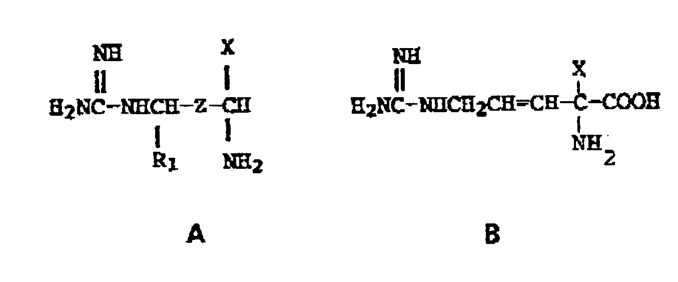 the pharmaceutically acceptable salts thereof, the individual optical isomers and mixtures thereof, wherein
X is -CH₂F, -CHF₂, and -CH=C=CH₂, Z is -CH₂-CH₂- or -CH=CH-,and R₁ is hydrogen, with the proviso that when Z is -CH₂CH₂-, X is -CH=C=CH₂,
which comprises contacting the corresponding intermediate compounds wherein the guanidino moiety is replaced by amino with a reactant providing substitution of an hydrogen atom of the amino group with a carboxamidine rest.

12. A process as in claim 11 wherein the reagent providing the substitution of the hydrogen atom of the amino group with the carboxamidine rest is 3,5-dimethylpyrazole-1-carboxamidine nitrate.

## Claims (Claims for the following Contracting State(s): AT, ES, GR)

1. A process preparing a compound of the formulae 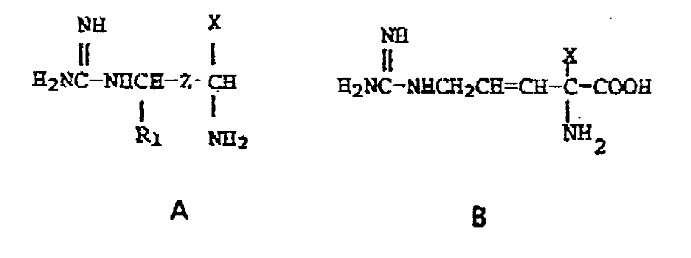 the pharmaceutically acceptable salts thereof, the individual optical isomers and mixtures thereof, wherein
X is -CH₂F, -CHF₂, and -CH=C=CH₂, Z is -CH₂-CH₂- or -CH=CH-,and R₁ is hydrogen, with the proviso that when Z is -CH₂CH₂-, X is -CH=C=CH₂,
which comprises contacting the corresponding intermediate compounds wherein the guanidino moiety is replaced by amino with a reactant providing substitution of an hydrogen atom of the amino group with a carboxamidine rest.

2. A process as in claim 1 wherein the reagent providing the substitution of the hydrogen atom of the amino group with the carboxamidine rest is 3,5-dimethylpyrazole-1-carboxamidine nitrate.

## Patentansprüche (Patentansprüche für folgende(n) Vertragsstaat(en): BE, CH, DE, FR, GB, IT, LI, LU, NL, SE)

1. Verwendung eines X-substituierten Arginins oder Agmatins der Formel 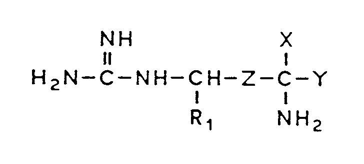 und ihrer pharmazeutisch verträglichen Salze, der einzelnen optischen Isomeren und ihrer racemischen Gemische, in der
R₁ ein Wasserstoffatom oder einen Rest der Formel -CH₃ bedeutet,
Z ein Rest der Formel -CH₂-CH₂- oder -CH=CH- ist,
X einen Rest der Formel -CH₂F, -CHF₂, -CHCl₂, -CHClF, -C≡CH oder -CH=C=CH₂ bedeutet,
Y ein Wasserstoffatom oder ein Rest der Formel -COOR ist, wobei R ein Wasserstoffatom oder einen (C₁₋₁₈)-Alkylrest bedeutet,
zur Herstellung eines Arzneimittels zur Behandlung der Chagas-Krankheit.

2. Verwendung einer Verbindung gemäß Anspruch 1, wobei X ein Halomethylrest ist.

3. Verwendung einer Verbindung gemäß Anspruch 1, wobei X eine Fluormethylgruppe ist.

4. Verwendung einer Verbindung gemäß Anspruch 1, wobei X eine Difluormethylgruppe ist.

5. Verwendung einer Verbindung gemäß Anspruch 1, die ein Argininderivat ist.

6. Verwendung einer Verbindung gemäß Anspruch 1, die ein Agmatinderivat ist.

7. Verbindungen der Formeln 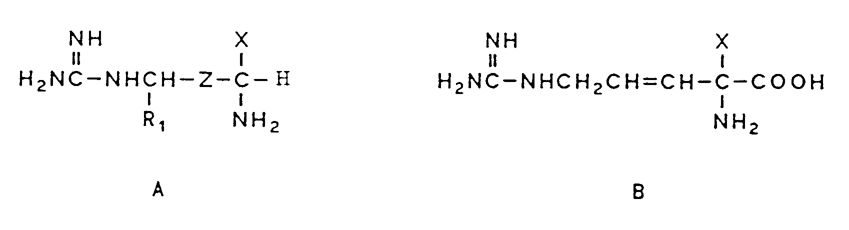 ihre pharmazeutisch verträglichen Salze, die einzelnen optischen Isomere und ihre Gemische, in denen
X einen Rest der Formel -CH₂F, -CHF₂ und -CH=C=CH₂ bedeutet,
Z ein Rest der Formel -CH₂-CH₂- oder -CH=CH- ist, und
R₁ ein Wasserstoffatom bedeutet, mit der Maßgabe, daß X einen Rest der Formel -CH=C=CH₂ bedeutet, falls Z ein Rest der Formel -CH₂CH₂- ist.

8. Verbindung nach Anspruch 7, wobei X einen Rest der Formel -CH₂F oder -CHF₂ bedeutet.

9. Verbindung nach Anspruch 7, wobei X einen Rest der Formel -CH=C=CH₂ bedeutet.

10. Verbindungen nach Anspruch 7, wobei die Doppelbindung in ihrer trans-Konfiguration vorliegt.

11. Verfahren zur Herstellung einer Verbindung einer der Formeln 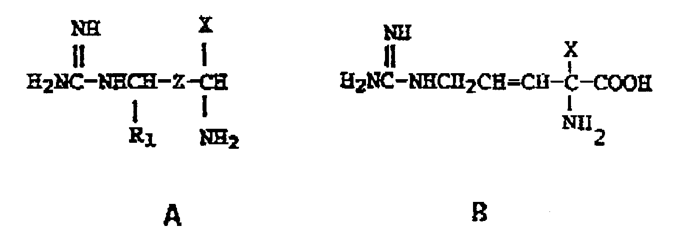 ihrer pharmazeutisch verträglichen Salze, ihrer einzelnen optischen Isomere und deren Gemische, in denen
X ein Rest der Formel -CH₂F, -CHF₂ und -CH=C=CH₂ bedeutet, Z ein Rest der Formel -CH₂-CH₂- oder -CH=CH- ist und R₁ ein Wasserstoffatom bedeutet, mit der Maßgabe, daß X ein Rest der Formel -CH=C=CH₂ bedeutet, falls Z ein Rest der Formel -CH₂CH₂- ist, umfassend das Zusammenbringen der entsprechenden Zwischenprodukte, in denen die Guanidinoeinheit durch eine Aminogruppe ersetzt ist, mit einem Umsetzungspartner, der den Ersatz eines Wasserstoffatoms der Aminogruppe durch einen Carboxamidinrest gewährleistet.

12. Verfahren nach Anspruch 11, wobei der Umsetzungspartner, der den Ersatz des Wasserstoffatoms der Aminogruppe durch einen Carboxamidinrest gewährleistet, 3,5-Dimethylpyrazol-1-carboxamidinnitrat ist.

## Patentansprüche (Patentansprüche für folgende(n) Vertragsstaat(en): AT, ES, GR)

1. Verfahren zur Herstellung einer Verbindung einer der Formeln 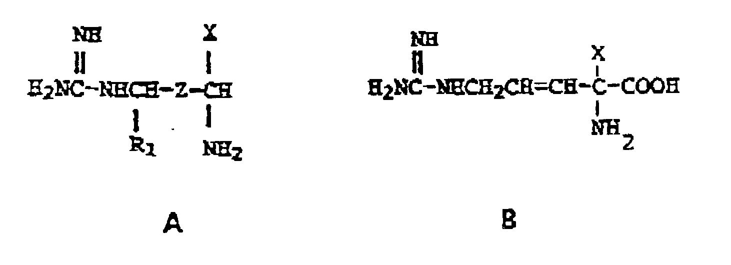 ihrer pharmazeutisch verträglichen Salze, ihrer einzelnen optischen Isomere und deren Gemische, in denen
X ein Rest der Formel -CH₂F, -CHF₂ und -CH=C=CH₂ bedeutet, Z ein Rest der Formel -CH₂-CH₂- oder -CH=CH- ist und R₁ ein Wasserstoffatom bedeutet, mit der Maßgabe, daß X ein Rest der Formel -CH=C=CH₂ bedeutet, falls Z ein Rest der Formel -CH₂CH₂- ist, umfassend das Zusammenbringen der entsprechenden Zwischenprodukte, in denen die Guanidinoeinheit durch eine Aminogruppe ersetzt ist, mit einem Umsetzungspartner, der den Ersatz eines Wasserstoffatoms der Aminogruppe durch einen Carboxamidinrest gewährleistet.

2. Verfahren nach Anspruch 1, wobei der Umsetzungspartner, der den Ersatz des Wasserstoffatoms der Aminogruppe durch einen Carboxamidinrest gewährleistet, 3,5-Dimethylpyrazol-1-carboxamidinnitrat ist.

## Revendications (Revendications pour l'(les) Etat(s) contractant(s) suivant(s): BE, CH, DE, FR, GB, IT, LI, LU, NL, SE)

1. Utilisation d'une arginine ou d'une agmatine X-substituée de formule : 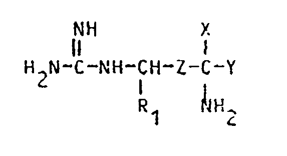 dans laquelle
R₁ est H ou CH₃,
Z est -CH₂-CH₂- ou -CH=CH-,
X est -CH₂F, -CHF₂, -CHCl₂, -CHClF, -C≡CH ou -CH=C=CH₂,
Y est H ou COOR où R est H ou un groupe alkyle en C₁-C₁₈,
et de leurs sels acceptables en pharmacie, de leurs isomères optiques isolés et de leurs mélanges racémiques pour préparer un médicament pour le traitement de la maladie de Chagas.

2. Utilisation d'un composé selon la revendication 1, dans lequel X est un groupe halogénométhyle.

3. Utilisation d'un composé selon la revendication 1, dans lequel X est un groupe fluorométhyle.

4. Utilisation d'un composé selon la revendication 1, dans lequel X est un groupe difluorométhyle.

5. Utilisation d'un composé selon la revendication 1 qui est un dérivé d'arginine.

6. Utilisation d'un composé selon la revendication 1 qui est un dérivé d'agmatine.

7. Composés de formules 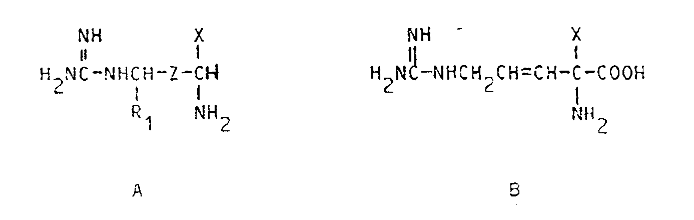 et de leurs sels acceptables en pharmacie, de leurs isomères optiques isolés et de leurs mélanges, dans lesquelles
X est -CH₂F, -CHF₂ ou -CH=C=CH₂,
Z est -CH₂-CH₂- ou -CH=CH- et
R₁ est l'hydrogène, avec la condition que lorsque Z est -CH₂CH₂-, X est -CH=C=CH₂.

8. Un composé selon la revendication 7, dans lequel X est -CH₂F ou -CHF₂.

9. Un composé selon la revendication 7, dans lequel X est -CH=C=CH₂.

10. Composés selon la revendication 7, dans lesquelles la double liaison est dans sa configuration trans.

11. Procédé de préparation d'un composé de formules 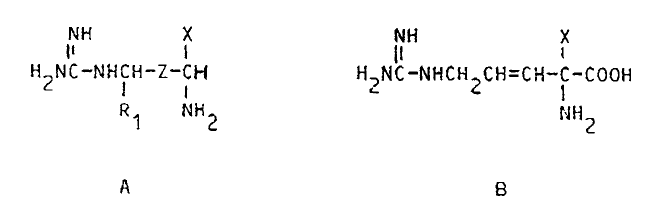 de ses sels pharmaceutiquement acceptables, de ses différents isomères optiques et de leurs mélanges,
formules dans lesquelles,
X est -CH₂F, -CHF₂ au -CH=C=CH₂,
Z est -CH₂-CH₂- ou -CH=CH- et
R₁ est l'hydrogène, avec la condition que lorsque Z est -CH₂CH₂-, X est -CH=C=CH₂,
qui comprend la mise en contact des composés intermédiaires correspondants, dans lesquels le reste guanidino est remplacé par amino avec un réactif permettant le remplacement d'un atome d'hydrogène du groupe amino par un reste carboxamidine.

12. Procédé selon la revendication 11, dans lequel le réactif permettant le remplacement de l'atome d'hydrogène du groupe amino par le reste carboxamidine est le nitrate de 3,5-diméthylpyrazole-1-carboxamidine.

## Revendications (Revendications pour l'(les) Etat(s) contractant(s) suivant(s): AT, ES, GR)

1. Procédé de préparation d'un composé de formules 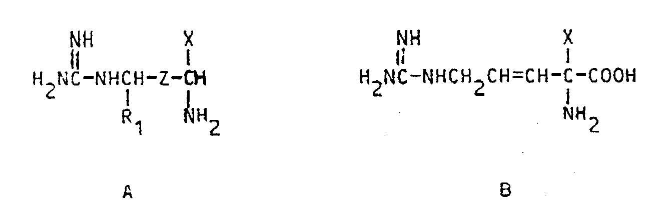 de ses sels pharmaceutiquement acceptables, de ses différents isomères optiques et de leurs mélanges,
formules dans lesquelles,
X est -CH₂F, -CHF₂ ou -CH=C=CH₂,
Z est -CH₂-CH₂- ou -CH=CH- et
R₁ est l'hydrogène, avec la condition que lorsque Z est -CH₂CH₂-, X est -CH=C=CH₂,
qui comprend la mise en contact des composés intermédiaires correspondants, dans lesquels le reste guanidino est remplacé par amino avec un réactif permettant le remplacement d'un atome d'hydrogène du groupe amino par un reste carboxamidine.

2. Procédé selon la revendications 1, dans lequel le réactif permettant le remplacement de l'atome d'hydrogène du groupe amino par le reste carboxamidine est le nitrate de 3,5-diméthylpyrazole-1-carboxamidine.
